# EUROPÄISCHE PATENTSCHRIFT

(11) **EP 0 506 833 B1**
(45) Veröffentlichungstag und Bekanntmachung des Hinweises auf die Patenterteilung: **31.08.1994**
(21) Anmeldenummer: 91902174.1
(22) Anmeldetag: 04.12.1990
(51) Int. Cl.: C12N 15/15, C12N 15/62, C12N 15/31, C07K 7/10, C12P 21/02

(54) **VERFAHREN ZUR HERSTELLUNG VON HIRUDIN**
METHOD OF PREPARATION OF HIRUDIN
PROCEDE DE PREPARATION DE L'HIRUDINE

(30) Priorität: 22.12.1989 DE 3942580
(43) Veröffentlichungstag der Anmeldung: 07.10.1992
(73) Patentinhaber: BASF Aktiengesellschaft, 67063 Ludwigshafen (DE)
(72) Erfinder: KOERWER, Wolfgang, D-67269 Grünstadt (DE)
(86) Internationale Anmeldenummer: EP9002084
(87) Internationale Veröffentlichungsnummer: WO9109946

(56) Entgegenhaltungen:
- EP-A- 0 211 299
- EP-A- 0 227 938
- EP-A- 0 229 998
- WO-A-84/03103
- WO-A-87/05934
- Biochemistry, Band 26, 1987, American Chemical Society, T. Moks et al. S. 5239-5244

## Beschreibung

Die vorliegende Erfindung betrifft ein Verfahren zur Herstellung von Hirudin mit Hilfe eines neuen Fusionsproteins.

Hirudin ist eine lange bekannte Substanz (vgl. Merck Index 1983, Nr. 4613), die antikoagulatorische Eigenschaften besitzt. Die Struktur des Hirudins ist ebenfalls bekannt (FEBS-Lett. 165, 180 (1984)). Hirudin kann an der Aminosäure Tyrosin in Position 63 sulfatiert sein.

Es ist bereits versucht worden, Hirudin gentechnisch herzustellen (FEBS-Lett. 202, 373 (1986), Biol. Chem. Hoppe-Seyler 367, 731 (1986)). Hierbei wurden allerdings schlechte Ausbeuten erzielt, weil das Hirudin in der Zelle offensichtlich proteolytisch nicht stabil oder die Expression sehr schwach war. Die Expression als Fusionsprotein brachte zwar höhere Rohausbeuten, doch ist hier die Aufreinigung technisch sehr aufwendig (DNA 5, 511 (1986)).

Es wurde nun gefunden, daß man Hirudinpeptide mit guten Ausbeuten und einfacher Aufreinigung über ein Fusionsprotein der Formel

X-Y-Hirudinpeptid

herstellen kann, worin
- X: Protein A oder eins seiner aktiven Polypeptidfragmente ist und
- Y: Met oder eine Oligopeptidsequenz darstellt, die sich an der Verbindungsstelle zum Hirudin oder in dessen Nähe Spalten läßt.

Die Bezeichnung Hirudinpeptid schließt Hirudin, am N-Terminus um 1-3 Aminosäuren verlängerte oder verkürzte Hirudine und Hirudinpeptide mit Hirudinaktivität ein.

Protein A ist ein bekanntes Protein (vgl. EP 135 532). Unter der Bezeichnung "aktives Polypeptidfragment" des Protein A sind solche Protein A-Fragmente zu verstehen, die noch die Fähigkeit besitzen, an Immunglobulin zu binden (vgl. Biochemistry 20, 2361-96 (1981) und Eur. J. Biochem. 156, 637-43 (1986)).

Y ist Met oder eine Oligopeptidsequenz, die weder in X noch im Hirudin vorliegt und die sich an der Verbindungsstelle zum Hirudin oder in dessen Nähe enzymatisch oder chemisch spalten läßt. Im einfachsten Fall besteht Y aus der Aminosäure Methionin.

Die Spaltung des Fusionsproteins kann nach bekannten Methoden durchgeführt werden. Ist Y ein Methionin-Rest, so kann die Spaltung mit Hilfe von Bromcyan erfolgen, wobei X-Homoserin und Hirudin freigesetzt werden. Wenn Y die Sequenz Asp-Pro enthält, ist es möglich, durch Säureeinwirkung zwischen diesen Aminosäuren zu Spalten. In diesem Fall verbleibt der Asp-Rest am Carboxylterminus des Protein A und Pro am N-Terminus des Hirudins. Weiter kommen unter anderen folgende Oligopeptidsequenzen für Y in Betracht: Asp↓Gly (Spaltung durch Hydroxylamin), Pro-Phe-His-Leu↓Leu (enzymatische Spaltung durch Renin), Ile-Glu-Gly-Arg↓Hirudin (enzymatische Spaltung durch Faktor VIIIa).

Je nach verwendetem Oligopeptid Y wird also entweder das Hirudin bzw. Hirudinpeptid freigesetzt, oder es entstehen Hirudin bzw. Hirudinpeptide, die verlängert sind.

Das Hirudinpeptid kann die usprünglich publizierte Aminosäuresequenz aufweisen (siehe FEBS-Lett. 165, 180 (1984) oder die Sequenz eines der inzwischen aufgefundenen Isohirudine (FEBS-Lett. 255, 105-110, 1989) oder künstlich hergestellter Mutanten mit Hirudinaktivität besitzen.

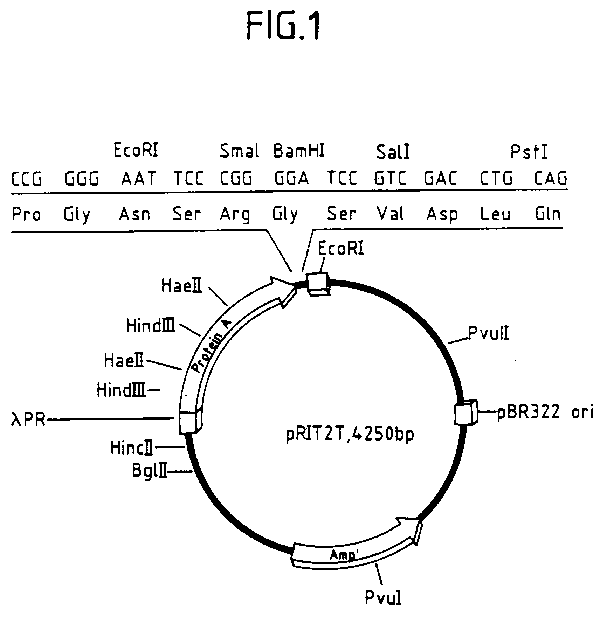

Als besonders vorteilhaft hat sich das Fusionsprotein erwiesen, das durch Ersatz des 388 bp großen N-terminalen Protein A-Fragments aus dem kommerziell erhältlichen Vektor pRIT 2T (Pharmacia, Best.Nr. 27-4808-01, Fig. 1) durch einen 97 bp großen synthetischen Adaptor (Sequenz 1) entstand. Die DNA-Sequenz sowie die dazugehörende Aminosäuresequenz zeigt die Sequenzübersicht 7. Die Aminosäuren 1-163 stellen das Protein A-Peptid X dar, die Aminosäuren 164-175 das Oligopeptid Y und die Aminosäuren 176-230 die Hirudinsequenz.

Die Fusionsproteine besitzen folgende herausragende Eigenschaften:
1) Sie lassen sich in E.coli sehr hoch exprimieren.
2) Sie sind proteolytisch sehr stabil.
3) Sie fallen in E.coli in löslicher Form an.
4) Sie sind thermisch sehr stabil (bis 80°C).
5) Sie binden an IgG-Sepharose ausschließlich über ihren Protein A-Teil.

Das Fusionsprotein des Beispiels 1 besitzt darüber hinaus folgende Vorteile:
a) Durch Spaltung mit BrCN entsteht reifes Hirudin ohne zusätzliche N-terminale Aminosäure.
b) Da das Methionin im Spaltpeptid das einzige Methionin im Fusionsprotein ist, entstehen bei der Spaltung zwei Fragmente. Von diesen kann der Fusionspartner vom Hirudin durch IgG-Affinitätschromatographie abgetrennt werden.

### Beispiel 1

### Herstellung des Expressionsplasmids

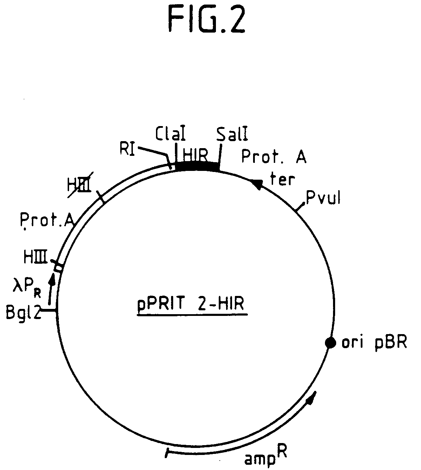

a) Konstruktion des Vektors
   Der Protein A-Vektor pRIT 2T (Figur 1) ist kommerziell erhältlich und ausführlich beschrieben (Pharmacia Bestell-Nr. 27-4808-01).
   Dieser Vektor wurde wie folgt modifiziert: Er wurde mit der Restriktionsendonuklease Hind III gespalten. Das größere Fragment (Vektor) wurde durch Elektroelution aus einem Agarosegel isoliert. In diesen Vektor wurden die komplementären Oligonukleotide Koe 1/2 (Sequenz 1) einligiert. Das entstandene chimäre Plasmid wurde in den Lambda-Lysogenstamm N 4830-1 (Pharmacia Best.Nr. 27-4808-01) transformiert. Der Klon mit der korrekten Orientierung der Oligonukleotide wurde mit Hilfe einer Hind III/EcoRI Restriktionskartierung aus den möglichen Rekombinanten herausgesucht und durch DNA-Sequenzierung überprüft. Dieses Expressionsplasmid wurde pRIT 2TA genannt.
b) Einsetzen eines synthetischen Hirudingens mit Adapter
   Die pRIT 2TA-DNA wurde mit EcoRI und SalI geschnitten und das größere DNA-Fragment (a) mit Hilfe der Elektroelution aus einem Agarosegel isoliert.
   Ein synthetisches Hirudingen (Sequenz 6) wurde mit Hilfe eines DNA-Synthesegeräts (Applied Biosystems, Modell 380A) hergestellt. Dazu wurden 4 Oligonukleotide (Koe 3-Koe 6) angefertigt (Sequenzen 2-5). Die Oligonukleotide wurden kinasiert und mit EcoRI/SalI linearisierten Plasmid pUC 18 ligiert. Die Konstruktion wurde mit Hilfe einer DNA-Sequenzierung überprüft. Aus diesem chimären Plasmid (pUC 18-Hir) wurde das Hirudingen (b) inklusive Adapter mit EcoRI herausgeschnitten und über Agarosegelelektrophorese und Elektroelution isoliert. Das synthetische Hirudingen beinhaltet neben zwei Stop-Kodons am 3'-Ende und der SalI-Erkennungsstelle eine Adaptersequenz, die das Hirudingen unter Beibehaltung des Leserasters an den Protein A-Fusionspartner über die EcoRI-Spaltstelle anknüpft.
   Die isolierten DNA-Fragmente a und b wurden miteinander ligiert. Es entstand so der Protein A-Hirudin Expressionsvektor pRIT2TA-Hir (Fig. 2).

### Beispiel 2

### Expression des Fusionsproteins

Das Expressionsplasmid pRIT 2TA-Hir wurde in den lysogenen Stamm E.coli N 4830-1 (Pharmacia Bestell-Nr. 27-4808-01) transformiert. Dieser Stamm enthält chromosomal den thermosensitiven Lambda-Repressor CI 857.

In einem 1l Erlenmeyerkolben mit Schikanen wurden 100 ml MIM-Medium (MIM = 32 g Trypton, 20 g Hefeextrakt, 6 g Na₂HPO₄, 3 g KH₂PO₄, 0,5 g NaCl, 1 g NH₄Cl pro Liter und 0,1 mM MgSO₄ sowie 0,001 mM FeCl₃) sterilisiert und Ampicillin (ad 100 µg/ml) zugegeben. Das Medium wurde mit 1 ml einer frischen Über-Nacht-Kultur des Stammes pRIT 2TA-Hir/N 4830-1 angeimpft und unter Schütteln so lange bei 28°C bebrütet, bis die Absorption bei 550 nm 0,6 betrug. Dann wurden 100 ml 65°C warmes frisches MIM/amp-Medium zugegeben und weitere 4 h bei 42°C bebrütet. In dieser Zeit wurde das gewünschte Fusionsprotein synthetisiert. Durch Zugabe von Lysozym zu 75 mg/l und Inkubation (3h, 37°C) wurde die Zellwand enzymatisch entfernt. Die Zellen konnten dann mechanisch (Manton-Gaulin Presse, Einfrierzyklus, heftiges Rühren), durch einen Hitzeschock bis 80°C oder eine hypotone Lyse aufgeschlossen und das lösliche Fusionsprotein ins Medium freigesetzt werden.

### Beispiel 3

### Reinigung des Fusionsproteins

Die Zellbruchstücke wurden durch Zentrifugation entfernt und der klare überstand über eine IgG-Sepharose-Säule (IgG-Sepharose® 6 ''Fast Flow, Pharmacia, Best.Nr. 17-0969-01) gepumpt. Die Lagerung des Säulenmaterials, Vorbereitung und Auslegung der Säule, Auftragsbedingungen und Flußraten richteten sich nach den Angaben des Herstellers. So wurde für 6 l überstand ein Gelbett von 200 ml und eine Flußrate von ca. 3 l/h verwendet. Bei diesem Schritt wurde das Fusionsprotein über seinen IgG-bindenden Protein A-Teil reversibel an die Gelmatrix gebunden (Ausbeute ca. 95 %). Nach dem Auftrag wurde die Säule mit 10 Bettvolumina TST (50 mM Tris-HCl pH 7,6; 150 mM NaCl und 0,05 % Tween®20) gewaschen. Die Elution erfolgte mit 0,5 M Acetatpuffer pH 2,8.

### Beispiel 4

### Spaltung des Fusionsproteins

Das Säuleneluat aus Beispiel 3 wurde lyophilisiert und bei -20°C gelagert. Zur Spaltung wurde es in 70 %iger Ameisensäure zu einer Proteinkonzentration von ca. 25 g/l aufgenommen. Nach Spülen mit Argon wurde zur Abspaltung des Hirudins 1 g BrCN als Feststoff pro g Fusionsprotein zugegeben. Die Spaltung erfolgte unter Argon bei 37°C in ca. 4 h. Das überschüssige Bromcyan, das Lösungsmittel und weitere flüchtige Bestandteile wurden durch Lyophilisation enfernt. Anschließend wurde dreimal mit Wasser gewaschen.

### Beispiel 5

### Renaturierung und Reinigung des Hirudins

Das Lyophilisat wurde zu 1-100 mg/ml Protein in 6 M Guanidin-HCl, 0,1 M Tris/HCl pH 8,5, 0,2 M DTT aufgenommen. Nach einer Inkubationszeit von 2 h wurde die Probe durch G-10-Ausschlußchromatographie (äquilibriert mit 10 mM HCl) entsalzt. Die entsalzte Probe wurde 1 : 20 in 0,1 M Tris/HCl, 5 mM GSH/0,5 mM GSSG, 1 mM EDTA, pH 8,7 verdünnt und 1 h inkubiert (GSH ist reduziertes und GSSG oxidiertes Glutathion). Durch diese Behandlung stieg die spezifische Aktivität des Hirudins um den Faktor 3-5. Nach Einstellen des pH-Wertes auf pH 7,6 mit HCl, Zugabe von NaCl auf 150 mM und Tween®20 auf 0,05 % wurde die Chromatografie über IgG-Sepharose wiederholt (Beispiel 3). Während der Protein A-Fusionspartner und ungespaltenes Fusionsprotein an die Säule banden, befand sich das aktive Hirudin mit einer Reinheit von > 90 % im Durchlauf. Es konnte durch klassische proteinchemische Verfahren bis zur klinischen Reinheit weiter aufgereinigt werden.

Sequenzübersicht

Sequenz 1:

Hind III
Ser Asn Asn Phe Asn Lys Glu Gln Gln Asn Ala Phe
Koe 1 5'-A GCT TCT AAC AAT TTC AAC AAA GAA CAA CAA AAT GCT TTC
Koe 2 3'-AGA TTG TTA AAG TTG TTT CTT GTT GTT TTA CGA AAG

Tyr Glu Ile Leu His Leu Pro Asn Leu Asn Glu Glu Gln Arg Asn Gly
TAT GAA ATC TTG CAT TTA CCT AAC TTA AAC GAA GAA CAA CGC AAT GGT
ATA CTT TAG AAC GTA AAT GGA TTG AAT TTG CTT CTT GTT GCG TTA CCA

Hind III
Phe Ile Gln
TTC ATC CAG-3'
AAG TAG GTC TCG A-5'

Sequenz 2:

Koe 3: 5'-AATTCAAAAA CCAAACCGCG TATCAAAACC ATGGTTGTTT ACACTGACTG CACTGAATCC
GGTCAGAACC TGTGCCTGTG CGAAGGCTCT AACGTTTGCG GCCAGGGCAA CAAATGCATC
CTGGGCTCT-3'

Sequenz 3:

Koe 4: 5'-GACGGCGAAA AAAACCAGTG CGTTACTGGC GAAGGTACCC CGAAACCGCA GTCTCACAAC
GACGGCGACT TCGAAGAAAT CCCGGAAGAA TACCTGCAGT AATAGG-3'

Sequenz 4:

Koe 5: 5'-TCGACCTATT ACTGCAGGTA TTCTTCCGGG ATTTCTTCGA AGTCGCCGTC GTTGTGAGAC
TGCGGTTTCG GGGTACCTTC GCCAGTAACG CACTGGTTTT TTTCGCCGTC AGAGCCCAGG
ATGCATTT-3'

Sequenz 5:

Koe 6: 5'-GTTGCCCTGG CCGCAAACGT TAGAGCCTTC GCACAGGCAC AGGTTCTGAC CGGATTCAGT
GCAGTCAGTG TAAACAACCA TGGTTTGATA CGCGGTTTGG TTTTTG-3'

Sequenz 6:

Eco RI
5'-AATTCAAAAA CCAAACCGCG TATCAAAACC ATGGTTGTTT ACACTGACTG CACTGAATCC
3'-GTTTTT GGTTTGGCGC ATAGTTTTGG TACCAACAAA TGTGACTGAC GTGACTTAGG

GGTCAGAACC TGTGCCTGTG CGAAGGCTCT AACGTTTGCG GCCAGGGCAA CAAATGCATC
CCAGTCTTGG ACACGGACAC GCTTCCGAGA TTGCAAACGC CGGTCCCGTT GTTTACGTAG

CTGGGCTCTG ACGGCGAAAA AAACCAGTGC GTTACTGGCG AAGGTACCCC GAAACCGCAG
GACCCGAGAC TGCCGCTTTT TTTGGTCACG CAATGACCGC TTCCATGGGG CTTTGGCGTC

SalI
TCTCACAACG ACGGCGACTT CGAAGAAATC CCGGAAGAAT ACCTGCAGTA ATAGG-3'
AGAGTGTTGC TGCCGCTGAA GCTTCTTTAG GGCCTTCTTA TGGACGTCAT TATCCAGCT-5'

Sequenz 7:

5 10 15
Met Glu Gln Arg Ile Thr Leu Lys Glu Ala Ser Asn Asn Phe Asn Lys
ATG GAA CAA CGC ATA ACC CTG AAA GAA GCT TCT AAC AAT TTC AAC AAA 48
TAC CTT GTT GCG TAT TGG GAC TTT CTT CGA AGA TTG TTA AAG TTG TTT 48

20 25 30
Glu Gln Gln Asn Ala Phe Tyr Glu Ile Leu His Leu Pro Asn Leu Asn
GAA CAA CAA AAT GCT TTC TAT GAA ATC TTG CAT TTA CCT AAC TTA AAC 96
CTT GTT GTT TTA CGA AAG ATA CTT TAG AAC GTA AAT GGA TTG AAT TTG 96

35 40 45
Glu Glu Gln Arg Asn Gly Phe Ile Gln Ser Leu Lys Asp Asp Pro Ser
GAA GAA CAA CGC AAT GGT TTC ATC CAG AGC TTA AAA GAT GAC CCA AGC 144
CTT CTT GTT GCG TTA CCA AAG TAG GTC TCG AAT TTT CTA CTG GGT TCG 144

50 55 60
Glu Ser Ala Asn Leu Leu Ser Glu Ala Lys Lys Leu Asn Glu Ser Gln
CAA AGT GCT AAC CTA TTG TCA GAA GCT AAA AAG TTA AAT GAA TCT CAA 192
GTT TCA CGA TTG GAT AAC AGT CTT CGA TTT TTC AAT TTA CTT AGA GTT 192

65 70 75 80
Ala Pro Lys Ala Asp Asn Lys Phe Asn Lys Glu Gln Gln Asn Ala Phe
GCA CCG AAA GCG GAT AAC AAA TTC AAC AAA GAA CAA CAA AAT GCT TTC 240
CGT GGC TTT CGC CTA TTG TTT AAG TTG TTT CTT GTT GTT TTA CGA AAG 240

85 90 95
Tyr Glu Ile Leu His Leu Pro Asn Leu Asn Glu Glu Gln Arg Asn Gly
TAT GAA ATC TTA CAT TTA CCT AAC TTA AAC GAA GAA CAA CGC AAT GGT 288
ATA CTT TAG AAT GTA AAT GGA TTG AAT TTG CTT CTT GTT GCG TTA CCA 288

100 105 110
Phe Ile Gln Ser Leu Lys Asp Asp Pro Ser Gln Ser Ala Asn Leu Leu
TTC ATC CAA AGC CTA AAA GAT GAC CCA AGC CAA AGC GCT AAC CTT TTA 336
AAG TAG GTT TCG GAT TTT CTA CTG GGT TCG GTT TCG CGA TTG GAA AAT 336

115 120 125
Ala Glu Ala Lys Lys Leu Asn Asp Ala Gln Ala Pro Lys Ala Asp Asn
GCA GAA GCT AAA AAG CTA AAT GAT GCT CAA GCA CCA AAA GCT GAC AAC 384
CGT CTT CGA TTT TTC GAT TTA CTA CGA GTT CGT GGT TTT CGA CTG TTG 384

130 135 140
Lys Phe Asn Lys Glu Gln Gln Asn Ala Phe Tyr Glu Ile Leu His Leu
AAA TTC AAC AAA GAA CAA CAA AAT GCT TTC TAT GAA ATT TTA CAT TTA 432
TTT AAG TTG TTT CTT GTT GTT TTA CGA AAG ATA CTT TAA AAT GTA AAT 432

145 150 155 160
Pro Asn Leu Thr Glu Glu Gln Arg Asn Gly Phe Ile Gln Ser Leu Lys
CCT AAC TTA ACT GAA GAA CAA CGT AAC GGC TTC ATC CAA AGC CTT AAA 480
GGA TTG AAT TGA CTT CTT GTT GCA TTG CCG AAG TAG GTT TCG GAA TTT 480

Sequenz 7:

165 170 175
Asp Asp Pro Gly Asn Ser Lys Thr Lys Pro Arg Ile Lys Thr Met Val
GAC GAT CCG GGG AAT TCA AAA ACC AAA CCG CGT ATC AAA ACC ATG GTT 528
CTG CTA GGC CCC TTA AGT TTT TGG TTT GGC GCA TAG TTT TGG TAC CAA 528

180 185 190
Val Tyr Thr Asp Cys Thr Glu Ser Gly Gln Asn Leu Cys Leu Cys Glu
GTT TAC ACT GAC TGC ACT GAA TCC GGT CAG AAC CTG TGC CTG TGC GAA 576
CAA ATG TGA CTG ACG TGA CTT AGG CCA GTC TTG GAC ACG GAC ACG CTT 576

185 190 195
Gly Ser Asn Val Cys Gly Gln Gly Asn Lys Cys Ile Leu Gly Ser Asp
GGC TCT AAC GTT TGC GGC CAG GGC AAC AAA TGC ATC CTG GGC TCT GAC 624
CCG AGA TTG CAA ACG CCG GTC CCG TTG TTT ACG TAG GAC CCG AGA CTG 624

200 205 210
Gly Glu Lys Asn Gln Cys Val Thr Gly Glu Gly Thr Pro Lys Pro Gln
GGC GAA AAA AAC CAG TGC GTT ACT GGC GAA GGT ACC CCG AAA CCG CAG 672
CCG CTT TTT TTG GTC ACG CAA TGA CCG CTT CCA TGG GGC TTT GGC GTC 672

215 220 225 230
Ser His Asn Asp Gly Asp Phe Glu Glu Ile Pro Glu Glu Tyr Leu Gln
TCT CAC AAC GAC GGC GAC TTC GAA GAA ATC CCG GAA GAA TAC CTG CAG 720
AGA GTG TTG CTG CCG CTG AAG CTT CTT TAG GGC CTT CTT ATG GAC GTC 720

Stop Stop
TAA TAG 726
ATT ATC 726

## Patentansprüche

1. Verfahren zur Herstellung von Hirudinpeptiden, dadurch gekennzeichnet, daß man ein Fusionsprotein der Formel
X-Y-Hirudinpeptid,
worin
X Protein A oder eines seiner aktiven Polypeptidfragmente ist und
Y Met oder eine Oligopeptidsequenz darstellt, die sich an der Verbindungsstelle zum Hirudin oder in dessen Nähe spalten läßt
in E. coli exprimiert und zwischen X und dem Hirudinpeptid spaltet.

## Claims

1. A process for the preparation of a hirudin peptide, which comprises a fusion protein of the formula
X-Y-hirudin peptide,
where X is protein A or one of its active polypeptide fragments, and Y is Met or an oligopeptide sequence which can be cleaved at the point of attachment to the hirudin or in the neighborhood thereof,
being expressed in E. coli and cleaved between X and the hirudin peptide.

## Revendications

1. Procédé de préparation d'hirudinopeptides, caractérisé en ce que l'on exprime une protéine de fusion de la formule
X-Y-Hirudinopeptide,
dans laquelle
X représente la protéine A ou un de ses fragments polypeptidiniques actifs et
Y représente Met ou une séquence oligopeptidique, qui se laisse scinder au site de liaison à l'hirudine ou à son voisinage,
dans E. coli et on la scinde entre X et l'hirudinopeptide.
